# EUROPEAN PATENT SPECIFICATION

(11) **EP 1 646 428 B1**
(45) Date of publication and mention of the grant of the patent: **09.04.2008**
(21) Application number: 04739191.7
(22) Date of filing: 12.05.2004
(51) Int. Cl.: A61K 8/43, A61K 8/49, A61Q 5/00

(54) **HAIR TREATMENT COMPOSITIONS**
HAARBEHANDLUNGSZUSAMMENSETZUNGEN
COMPOSITION DE TRAITEMENT DES CHEVEUX

(30) Priority: 29.05.2003 EP 03253383
(43) Date of publication of application: 19.04.2006
(73) Proprietor: UNILEVER PLC, London EC4P 4BQ (GB); UNILEVER N.V., 3013 AL Rotterdam (NL)
(72) Inventor: MAHADESHWAR, Anand Ramchandra, Bebington, Wirral Merseyside CH63 3JW (GB); SINGHAPUNTU, Siam, UNILEVER THAI HOLDINGS LIMITED, Ladyao, Jatujak, Bangkok, 10900 (TH)
(74) Representative: Tansley, Sally Elizabeth
(86) International application number: PCT/EP2004/005128
(87) International publication number: WO 2004/105705

(56) References cited:
- WO-A-01/85110
- US-A- 4 304 244
- US-A- 5 470 579
- US-A- 5 639 449
- PATENT ABSTRACTS OF JAPAN vol. 016, no. 518 (C-0999), 26 October 1992 (1992-10-26) & JP 04 193821 A (TAKEO KANEKO), 13 July 1992 (1992-07-13) cited in the application

## Description

### FIELD OF THE INVENTION

The invention relates to hair treatment compositions. The compositions are particularly suitable for straightening hair and decreasing its volume.

### BACKGROUND AND PRIOR ART

The use of xanthines in hair and skin pigmentation is described in US 5470579. US 4931066 discloses that xanthines can be used together with other compounds in hair dyeing.

Xanthines have also been claimed as being useful in the treatment of hair loss. For example, WO 85/05270, WO 84/04038 and WO 85/05272 all disclose hair loss treatments containing caffeine or theophylline as phosphodiesterase inhibitors. A hair nourishing lotion or shampoo containing caffeine and the hair loss treatment compound minoxidil is described in JP 04-193821.

WO 01/85110 discloses hair cosmetic compositions comprising guanidinium salts as active agents for Straightening hair.

The present application discloses formulations and processes for straightening hair. The invention has the further advantages that it prevents the hair frizzing and increasing in volume.

### DESCRIPTION OF THE INVENTION

In a first aspect, the present invention provides a hair treatment composition comprising:
i) a guanidinium salt selected from guanidinium carbonate, guanidinium sulphate or guanidinium phosphate; and
ii)a xanthine, having the following formula: 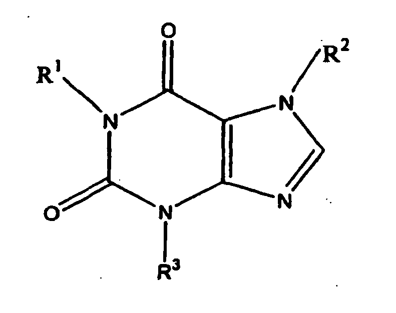
in which R¹ R² and R³ are independently selected from H, substituted or unsubstituted C₁-C₅ alkyl groups, substituted or unsubstituted C₂-C₅ alkenyl groups, aryl groups, arylalkyl groups or mixtures thereof.

A further aspect of the invention is the use of the above composition for straightening hair and decreasing its volume.

The invention also relates to a method of treating hair by applying to the hair the composition described above.

### Detailed Description of the Invention

### Guanidinium Salt

A guanidinium salt is present in formulations of the invention selected from guanidinium carbonate, guanidinium sulphate and guanidinium phosphate. Most preferred is guanidinium carbonate.

Guanidinium salts are best used at levels from 0.01wt% to 5wt% of the total formulation, more preferably at 0.01wt%w to 2 wt%, most preferably from 0.02 to 1 wt%.

### Xanthines

Xanthines (the term xanthines is taken as meaning xanthines and substituted xanthines) are compounds having formula I: 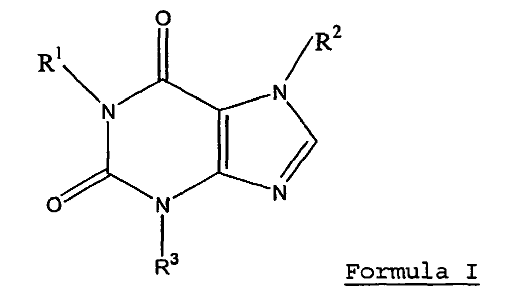 in which R¹, R² and R³ are independently selected from H, substituted or unsubstituted C₁-C₅ alkyl groups, substituted or unsubstituted C₂-C₅ alkenyl groups, aryl groups arylalkyl groups or mixtures thereof,. It is preferable if the substituted groups discussed above are amine or hydroxy groups.

It is advantageous if R¹, R² and R³ are H, C₁-C₅ alkyl, C₂-C₅ alkenyl or mixtures thereof. It is particularly preferred if R¹, R² and R³ are independently selected from H, methyl groups or mixtures thereof.

Most preferred are substituted xanthines such as caffeine, dyphylline, cafaminol theophylline, aminophylline and theobromine. Of these, caffeine is particularly preferred.

The xanthines may be in the form of a salt. The cosmetically acceptable salts of the xanthines of formula (I) are salts, which are non-toxic to humans in the context of the uses according to the invention. The nature of the salt will depend on the acidic or basic groups present in the xanthines which will depend, in turn, on their precise structural formula. Suitable acid addition salts include, for example, hydrochlorides, sulphates,phosphate, carboxylates (including acetates, citrates, tartarates, malates, malonates, maleates, lactates, succinates, and fumarates). Suitable base salts include, for example, ammonium salts and alkali metal salts (such as sodium and potassium salts). Suitable salts can be obtained by methods well-known to those skilled in the art.

Xanthines can be produced synthetically or extracted from natural products, such as plant extracts. For example, certain xanthines can be obtained from cacao beans, tea leaves and cola beans. Xanthines may be used in the present invention in substantially pure form, in the form of unpurified natural extracts, or as a mixture of substantially pure form and natural extract.

Xanthines may be used in the present invention singly or together with one or more other different xanthines.

The hair treatment composition of the invention preferably comprises from 0.01 to 10 wt% of xanthine/substituted xanthine of the total formulation, more preferably from 0.1 to 5 wt%.

The weight ratio of xanthine to guanidinium salt is preferably from 100:1 to 1:20, more preferably from 50:1 to 1:5, most preferably from 20:1 to 1:1.

### The disaccharide

The present invention may also comprises a disaccharide, preferably the disaccharide comprises of pentose or hexose sugars, more preferably the disaccharide comprises of two hexose units.

Disaccharides can be either reducing or non-reducing sugars. Non-reducing sugars are preferred.

The D(+) form of the sugars are preferred. Particularly preferred are trehalose and cellobiose or mixtures thereof. Trehalose is the most preferred disaccharide.

The level of disaccharides present in the total formulation is preferably from 0.01wt% to 10wt%, more preferably from 0.1wt% to 5wt%, most preferably from 0.2wt% to 2wt%.

### α-Hydroxy acids

The formulations of the invention may comprise an α - hydroxy acid. The hydroxy acid and/or its salt is preferably a bis (α -hydroxy acid) and/or its salt.
The α -hydroxy acid can comprise one or more carboxylic acid groups, at least one of these carboxylic acid groups should have an α -hydroxy group.

It is particularly preferred if the α -hydroxy acid and/or its salt if optically active is in the L-form such as those derived from natural sources. A particularly preferred α - hydroxy acid is tartaric acid and/or its salt.

The amount of α -hydroxy acid i) is from 0.1 to 20 wt% in the total formulation.

### Di-Acids

Di-acids having formula II are preferably present in the compositions of the present invention,

XOOC-(CH₂)ₙ-COOX Formula II

where n is an integer from 2 to 10 more preferably 2 to 6, most preferably where n equal to 2 or 4 (succinic acid and adipic acid respectively). X is H or an alkali metal cation, a particularly preferred alkali metal is sodium.

Diacids are best used at levels in the total formulation from 0.01 wt% to 5wt%, more preferably at levels from 0.1wt% to 2wt%.

The weight ratio of di-acid to α -hydroxy acid is 1:1 to 1:3 20, more preferably 1:5 to 1:15 .

The pH of the formulations of the invention are in the range from pH 3 to pH 6, more preferably used at pH 3-5.

A particularly preferred form of the invention comprises an a diacid as described above, a xanthine, a guanadine derivative, and a disaccharide. This combination of 5 ingredients gives particularly good results for volume control.

### Product Form

The final product form of hair treatment compositions according to the invention may suitably be, for example, conditioners, sprays, mousses, gels, waxes, lotions or shampoos. Particularly preferred product forms are post-wash conditioners (leave-in and rinse-off) and hair treatment products such as hair essences. Leave on formulations are most preferred.

### Conditioning Surfactant

Conditioner compositions usually comprise one or more conditioning surfactants, which are cosmetically acceptable and suitable for topical application to the hair.

Suitable conditioning surfactants are selected from cationic surfactants, used singly or in admixture.

Cationic surfactants useful in compositions of the invention contain amino or quaternary ammonium hydrophilic moieties, which are positively charged when, dissolved in the aqueous composition of the present invention.

The most preferred cationic surfactants for conditioner compositions of the present invention are monoalkyl quaternary ammonium compounds in which the alkyl chain length is C16 to C22.

Examples of suitable cationic surfactants include quaternary ammonium compounds, particularly trimethyl quaternary compounds.

Preferred quaternary ammonium compounds include cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these where the chloride is replaced by halogen, (e.g. , bromide), acetate, citrate, lactate, glycolate, phosphate nitrate, sulphate, or alkylsulphate. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese.

Salts of primary, secondary, and tertiary fatty amines are also suitable cationic surfactants.

In the conditioners of the invention, the level of cationic surfactant is preferably from 0.01 to 10, more preferably 0.05 to 5, most preferably 0.1 to 2 wt% of the total composition.

### Fatty Materials

Conditioner compositions of the invention preferably additionally comprise fatty materials. By "fatty material" is meant a fatty alcohol, an alkoxylated fatty alcohol, a fatty acid or a mixture thereof.

(Or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty alcohol material in conditioners of the invention is suitably from 0.01 to 15, preferably from 0.1 to 10, and more preferably from 0.1 to 5 wt%. The weight ratio of cationic surfactant to fatty alcohol is suitably from 10:1 to 1:10, preferably from 4:1 to 1:8, optimally from 1:1 to 1:7, for example 1:3.

### Suspending Agents

In a preferred embodiment, the hair treatment composition, especially if it is a shampoo composition, further comprises from 0.1 to 5 wt% of a suspending agent.

### Silicone Conditioning Agents

The compositions of the invention can contain, emulsified droplets of a silicone-conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

A further preferred class of silicones for inclusion in shampoos and conditioners of the invention are amino function.

The total amount of silicone is preferably from 0.01 to 10 %wt of the total composition more preferably from 0.3 to 5, most preferably 0.5 to 3 wt% is a suitable level.

### (ii) Non-silicone Oily Conditioning Components

Compositions according to the present invention may also comprise a dispersed, non-volatile, water-insoluble oily conditioning agent.

By "insoluble" is meant that the material is not soluble in water (distilled or equivalent) at a concentration of 0.1% (w/w), at 25°C.

Suitable oily or fatty materials are selected from hydrocarbon oils, fatty esters and mixtures thereof.

### Adjuvants

The compositions of the present invention may also contain adjuvants suitable for hair care. Generally such ingredients are included individually at a level of up to 2, preferably up to 1 wt% of the total composition.

Suitable hair care adjuvants, include amino acids, sugars and ceramides.

### Styling Polymers

The hair styling polymer is preferably present in the compositions of the invention in an amount of from 0.001% to 10% by weight, more preferably from 0.1% to 10% by weight, such as from 1% to 8% by weight.

Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

Examples of anionic hair styling polymers are:
copolymers of vinyl acetate and crotonic acid;
terpolymers of vinyl acetate, crotonic acid and a vinyl ester of an alpha-branched saturated aliphatic monocarboxylic acid such as vinyl neodecanoate;
copolymers of methyl vinyl ether and maleic anhydride (molar ratio about 1:1) wherein such copolymers are 50% esterified with a saturated alcohol containing from 1 to 4 carbon atoms such as ethanol or butanol;
acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate); glycols having from 1 to 6 carbon atoms (such as hydroxypropyl methacrylate and hydroxyethyl acrylate); styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.
The additional styling polymer may also contain grafted silicone, such as polydimethylsiloxane.

Specific examples of suitable anionic hair styling polymers are:
RESYN® 28-2930 available from National Starch (vinyl acetate/crotonic acid/vinyl neodecanoate copolymer);
ULTRAHOLD® 8 available from BASF (CTFA designation Acrylates/acrylamide copolymer);
the GANTREZ®ES series available from ISP Corporation esterified copolymers of methyl vinyl ether and maleic anhydride).

Other suitable anionic hair styling polymers include carboxylated polyurethanes. Carboxylated polyurethane resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and a polyurethane, having a CTFA designation PVP/polycarbamyl polyglycol ester. Suitable carboxylated polyurethane resins are disclosed in EP-A-0619111 and US Patent No. 5,000,955. Other suitable hydrophilic polyurethanes are disclosed in US Patent Nos. 3,822,238; 4,156,066; 4,156,067; 4,255,550; and 4,743,673.

Amphoteric hair styling polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/ acrylates/butylaminoethyl methacrylate copolymer) sold by the National Starch and Chemical Corporation.

Examples of nonionic hair styling polymers are homopolymers of N- vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-0240350 and cross-linked silicone gums such as those described in WO 96/31188.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat® FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

Examples of suitable naturally-derived polymers include shellac, alginates, gelatins, pectins, cellulose derivatives and chitosan or salts and derivatives thereof. Commercially available examples include Kytamer® (ex Amerchol) and Amaze® (ex National Starch).

Also suitable for use as additional styling polymers in the compositions of the invention are the ionic copolymers described in WO 93/03703, the polysiloxane-grafted polymers disclosed in WO 93/23446, the silicone-containing polycarboxylic acid copolymers described in WO 95/00106 or WO 95/32703, the thermoplastic elastomeric copolymers described in WO 95/01383, WO 95/06078, WO 95/06079 and WO 95/01384, the silicone grafted adhesive polymers disclosed in WO 95/04518 or WO 95/05800, the silicone macro-grafted copolymers taught in WO 96/21417, the silicone macromers of WO 96/32918, the adhesive polymers of WO 98/48770 or WO 98/48771 or WO 98/48772 or WO 98/48776, the graft polymers of WO 98/51261 and the grafted copolymers described in WO 98/51755.

### Cleansing Surfactant

If the product is in the from of a shampoo compositions it may comprise one or more anionic cleansing surfactants which are cosmetically acceptable and suitable for topical application to the hair.

The shampoo composition can optionally include co-surfactants, preferably an amphoteric, nonionic or zwitterionic surfactant.

The invention will now be further illustrated by the following, non-limiting Examples.

All percentages quoted are by weight based on total weight unless otherwise stated.

### EXAMPLES

### Examples A,B,C 1 to 4

Formulations were prepared having ingredients as shown in the following Table.

Examples of the invention are illustrated by a number, Comparative Examples are illustrated by a letter.

| INCI NAME | % active in final product | | | | | | |
|---|---|---|---|---|---|---|---|
| | **A** | **B** | **1** | **C** | **2** | **3** | **4** |
| Cetrimonium Chloride | 1.30 | 1.30 | 1.30 | 1.30 | 1.30 | 1.30 | 1.30 |
| Glyceryl Stearate | 0.60 | 0.60 | 0.60 | 0.60 | 0.60 | 0.60 | 0.60 |
| Hydroxyethylcellulose | 0.15 | 0.15 | 0.15 | 0.15 | 0.15 | 0.15 | 0.15 |
| Cetyl Acetate/ Acetilated Lanolin Alcohol | 0.40 | 0.40 | 0.40 | 0.40 | 0.40 | 0.40 | 0.40 |
| Cetearyl Alcohol | 5.00 | 5.00 | 5.00 | 5.00 | 5.00 | 5.00 | 5.00 |
| Dimethiconol/TEA-Dodecyl-benzenesulfonate | 1.30 | 1.30 | 1.30 | 1.30 | 1.30 | 1.30 | 1.30 |
| Methylparaben | 0.20 | 0.20 | 0.20 | 0.20 | 0.20 | 0.20 | 0.20 |
| Paraffinum Liquidum | 0.30 | 0.30 | 0.30 | 0.30 | 0.30 | 0.30 | 0.30 |
| Petrolatum | 0.20 | 0.20 | 0.20 | 0.20 | 0.20 | 0.20 | 0.20 |
| Glycerin | 1.00 | 1.00 | 1.00 | 1.00 | 1.00 | 1.00 | 1.00 |
| Fragrance | 0.40 | 0.40 | 0.40 | 0.40 | 0.40 | 0.40 | 0.40 |
| Citric Acid | 0.01 | 0.01 | 0.01 | 0.01 | 0.10 | 0.10 | 0.01 |
| Preservative | 0.17 | 0.17 | 0.17 | 0.17 | 0.17 | 0.17 | 0.17 |
| Tartaric acid | - | 1.50 | 1.50 | 1.50 | 1.50 - | | 1.50 |
| Trehalose | - | 0.50 | - | 0.50 | 0.50 | 0.50 | 0.50 |
| Caffeine | - | 0.50 | 0.50 | - | 0.50 | 0.50 | 0.50 |
| Adipic acid | - | 0.15 | 0.15 | 0.15 | - | 0.15 | 0.15 |
| Guanidine carbonate | - | - | 0.05 | 0.05 | 0.05 | 0.05 | 0.05 |
| Water | .. | .. | to | 100 | % | ... | ... |

| | | | | | | | |
|---|---|---|---|---|---|---|---|
| *Citric acid is used in the formulation to adjust the pH. | | | | | | | |

### Switch test protocol -

A 8gm 10inch Asian wavy hair switch was used. Three switches were used for each prototype. The hair switch was subjected to shampoo, conditioner and after rinsing on the damp hair leave-on conditioner was applied. The switches were allowed to dry overnight at 25C and 50 %RH overnight. Next day all the switches were combed twice to break the water forces.

Examples 1 to 4 gave superior straightening benefit to Comparative Examples A to C.

### Panel test protocol for Hair Volume -

A pair comparison panel test was carried out to quantify low volume. 12 panellists were used to measure low volume. Each prototype was compare against Example 4 to quantify low volume.

### Examples

### Hair Low Volume

| Prototype | No. of times the panellist preferred | Significance |
|---|---|---|
| 4 | 33 | Not Significant |
| 3 | 39 | |
| | | |

| Prototype | No. of times the panellist preferred | Significance |
|---|---|---|
| 4 | 45 | 95% Significant |
| C | 27 | |
| 4 | 45 | 95% Significant |
| 2 | 27 | |
| | | |

| Prototype | No. of times the panellist preferred | Significance |
|---|---|---|
| 4 | 45 | 95% Significant |
| 1 | 27 | |
| | | |

| Prototype | No. of times the panellist preferred | Significance |
|---|---|---|
| 4 | 58 | 99% Significant |
| B | 14 | |

### Hair Straightening

### Salon half head test -

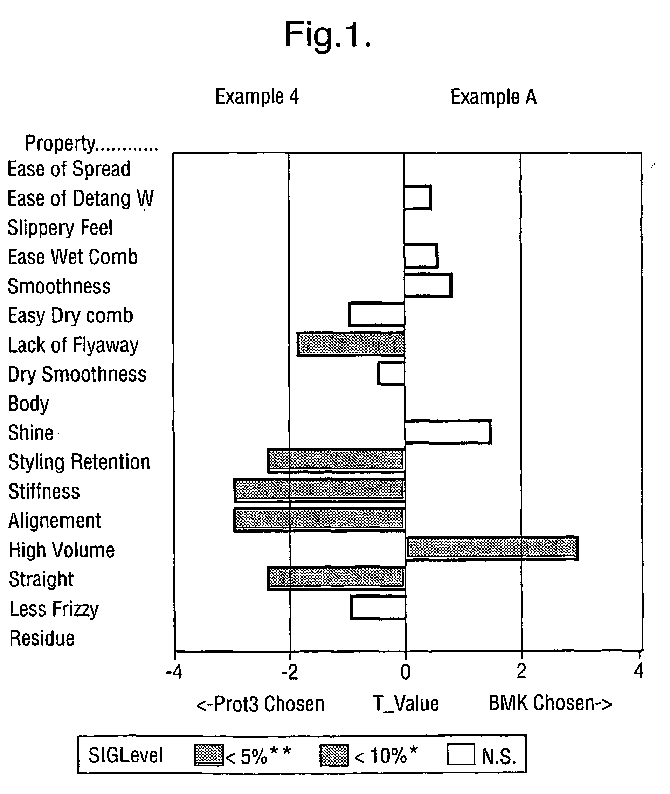

The salon test was carried out in Argentina. 12 wavy hair panellist were used for the test. The panellist hair (whole head) was subjected to shampoo and conditioner. Then the hair was divided in the middle and the Example 4 and Example A products were applied on respective half heads. The results of panellist assessment of various attributes are shown in Figure 1.

## Claims

1. A hair treatment composition comprising:
i) a guanidinium salt selected from guanidinium carbonate, guanidinium sulphate or guanidinium phosphate; and
ii)a xanthine, having the following formula: 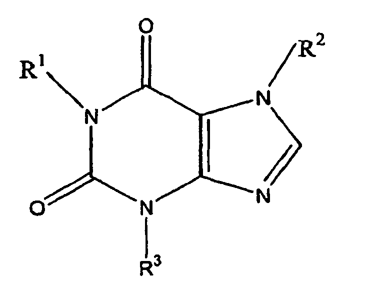
in which R¹, R² and R³ are independently selected from H, substituted or unsubstituted C₁-C₅ alkyl groups, substituted or unsubstituted C₂-C₅ alkenyl groups, aryl groups, arylalkyl groups or mixtures thereof.

2. A hair treatment composition according to claim 1 in which the a guanidinium salt is guanidinium carbonate.

3. A hair treatment composition according to claim 1 or claim 2 in which R¹, R² and R³ of the xanthine ii) are independently selected from H, C₁-C₅ alkyl groups, C₂-C₅ alkenyl or mixtures thereof.

4. A hair treatment composition according to any preceding claim in which R¹. R² and R³ of ii) are independently selected from H, methyl groups or mixtures thereof.

5. A hair treatment composition according to any preceding claim in which the xanthine ii) is caffeine.

6. A hair treatment composition according to any preceding claim further comprising a di-acid, its salt or mixtures thereof.

7. A hair treatment composition according to claim 6 in which the di-acid or its salt has the formula:
XOOC-(CH₂)ₙ-COOX
where n is an integer from 2 to 10 and X is H or an alkali metal cation.

8. A hair treatment composition according to claim 7 in which the di-acid is adipic acid.

9. A hair treatment composition according to any preceding claim further comprising a disaccharide.

10. A hair treatment composition according to any preceding claim in which the disaccharide has two hexose rings.

11. A hair treatment composition according to claim 9 in which the disaccharide is trehalose.

12. A hair treatment composition according to any preceding claim comprising, a xanthine, a guanidine derivative, a disaccharide and a C₂-C₆ diacid.

13. A hair treatment composition according to any preceding claim which comprises a cationic or silicone based conditioning agent.

14. A hair treatment composition according to any preceding claim which comprises an aqueous base.

15. Use of a composition according to any preceding claim for straightening the hair.

16. Use of a composition according to any preceding claim for reducing the volume of hair.

17. A method of treating hair by applying to the hair a composition according to any one of claims 1 to 14.

## Patentansprüche

1. Haarbehandlungszusammensetzung, umfassend:
i) ein Guanidiniumsalz, ausgewählt aus Guanidiniumcarbonat, Guanidiniumsulfat oder Guanidiniumphosphat; und
ii) ein Xanthin mit der nachstehenden Formel: 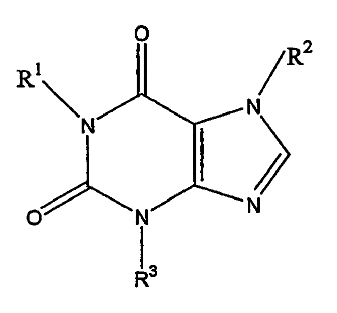
worin R¹, R² und R³ unabhängig aus H, substituierten oder unsubstituierten C₁-C₅-Alkylgruppen, substituierten oder unsubstituierten C₂-C₅-Alkenylgruppen, Arylgruppen, Arylalkylgruppen oder Gemischen davon ausgewählt sind.

2. Haarbehandlungszusammensetzung nach Anspruch 1, worin das Guanidiniumsalz Guanidiniumcarbonat ist.

3. Haarbehandlungszusammensetzung nach Anspruch 1 oder Anspruch 2, worin R¹, R² und R³ von dem Xanthin ii) unabhängig aus H, C₁-C₅-Alkylgruppen, C₂-C₅-Alkenyl oder Gemischen davon ausgewählt sind.

4. Haarbehandlungszusammensetzung nach einem vorangehenden Anspruch, worin R¹, R² und R³ von ii) unabhängig aus H, Methylgruppen oder Gemischen davon ausgewählt sind.

5. Haarbehandlungszusammensetzung nach einem vorangehenden Anspruch, worin das Xanthin ii) Koffein ist.

6. Haarbehandlungszusammensetzung nach einem vorangehenden Anspruch, weiterhin umfassend eine Di-Säure, ihr Salz oder Gemische davon.

7. Haarbehandlungszusammensetzung nach Anspruch 6, worin die Di-Säure oder ihr Salz die Formel aufweist:
XOOC - (CH₂)ₙ- COOX
worin n eine ganze Zahl von 2 bis 10 ist und X H oder ein Alkalimetall-Kation ist.

8. Haarbehandlungszusammensetzung nach Anspruch 7, worin die Di-Säure Adipinsäure ist.

9. Haarbehandlungszusammensetzung nach einem vorangehenden Anspruch, weiterhin umfassend ein Disaccharid.

10. Haarbehandlungszusammensetzung nach einem vorangehenden Anspruch, worin das Disaccharid zwei Hexoseringe aufweist.

11. Haarbehandlungszusammensetzung nach Anspruch 9, worin das Disaccharid Trehalose ist.

12. Haarbehandlungszusammensetzung nach einem vorangehenden Anspruch, umfassend ein Xanthin, ein Guanidin-Derivat, ein Disaccharid oder eine C₂-C₆-Di-Säure.

13. Haarbehandlungszusammensetzung nach einem vorangehenden Anspruch, die ein kationisches oder auf Silikon basierendes konditionierendes Mittel umfasst.

14. Haarbehandlungszusammensetzung nach einem vorangehenden Anspruch, die eine wässrige Base umfasst.

15. Verwendung einer Zusammensetzung nach einem vorangehenden Anspruch zum Glätten bzw. Entwirren des Haars.

16. Verwendung einer Zusammensetzung nach einem vorangehenden Anspruch zum Vermindern des Haarvolumens.

17. Verfahren zum Behandeln von Haar durch Auftragen einer Zusammensetzung nach einem der Ansprüche 1 bis 14 auf das Haar.

## Revendications

1. Composition pour le traitement des cheveux comprenant :
i) un sel de guanidinium choisi parmi le carbonate de guanidinium, le sulfate de guanidinium ou le phosphate de guanidinium ; et
ii) une xanthine, ayant la formule suivante : 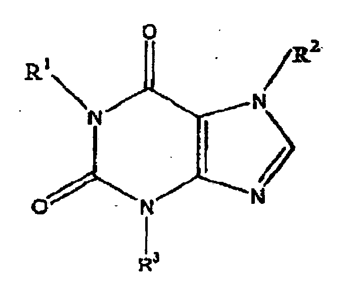
dans laquelle R¹, R² et R³ sont indépendamment choisis parmi H, des groupes alkyles en C₁ à C₅ substitués ou non substitués, des groupes alcényles en C₂ à C₅ substitués ou non substitués, des groupes aryles, des groupes arylalkyles ou des mélanges de ceux-ci.

2. Composition pour le traitement des cheveux selon la revendication 1, dans laquelle ledit sel de guanidinium est un carbonate de guanidinium.

3. Composition pour le traitement des cheveux selon la revendication 1 ou la revendication 2, dans laquelle R¹, R² et R³ de la xanthine ii) sont indépendamment choisis parmi H, des groupes alkyles en C₁ à C₅, des groupes alcényles en C₂ à C₅ ou des mélanges de ceux-ci.

4. Composition pour le traitement des cheveux selon l'une quelconque des revendications précédentes, dans laquelle R¹, R² et R³ de ii) sont indépendamment choisis parmi H, des groupes méthyles ou des mélanges de ceux-ci.

5. Composition pour le traitement des cheveux selon l'une quelconque des revendications précédentes, dans laquelle la xanthine ii) est la caféine.

6. Composition pour le traitement des cheveux selon l'une quelconque des revendications précédentes, comprenant en outre un diacide, un de ses sels ou des mélanges de ceux-ci.

7. Composition pour le traitement des cheveux selon la revendication 6, dans laquelle le diacide ou son sel a pour formule :
XOOC- (CH₂)ₙ-COOX
où n est un entier de 2 à 10 et X est H ou un cation d'un métal alcalin.

8. Composition pour le traitement des cheveux selon la revendication 7, dans laquelle le diacide est l'acide adipique.

9. Composition pour le traitement des cheveux selon l'une quelconque des revendications précédentes, comprenant en outre un disaccharide.

10. Composition pour le traitement des cheveux selon l'une quelconque des revendications précédentes, dans laquelle le disaccharide comporte deux cycles hexoses.

11. Composition pour le traitement des cheveux selon la revendication 9, dans laquelle le disaccharide est le tréhalose.

12. Composition pour le traitement des cheveux selon l'une quelconque des revendications précédentes, comprenant une xanthine, un dérivé de la guanidine, un disaccharide et un diacide en C₂ à C₆.

13. Composition pour le traitement des cheveux selon l'une quelconque des revendications précédentes comprenant un agent de conditionnement cationique ou à base de silicone.

14. Composition pour le traitement des cheveux selon l'une quelconque des revendications précédentes, comprenant une base aqueuse.

15. Utilisation d'une composition selon l'une quelconque des revendications précédentes pour défriser les cheveux.

16. Utilisation d'une composition selon l'une quelconque des revendications précédentes pour réduire le volume des cheveux.

17. Procédé de traitement des cheveux par application sur les cheveux d'une composition selon l'une quelconque des revendications 1 à 14.
